# EUROPEAN PATENT APPLICATION

(11) **EP 1 420 065 A1**
(43) Date of publication of application: **19.05.2004**
(21) Application number: 02746156.5
(22) Date of filing: 01.08.2002
(51) Int. Cl.: C12N 7/02, C12N 15/90, A61K 35/76, A61K 48/00

(54) **PROCESS FOR PRODUCING INACTIVATED VIRUS ENVELOPE**

(30) Priority: 02.08.2001 JP 2001235313
(71) Applicant: Anges MG Inc., Toyonaka-shi, Osaka 560-0082 (JP)
(72) Inventor: KANEDA, Yasufumi, c/o AnGes MG, Inc., Toyonaka-shi, Osaka 560-0082 (JP)
(74) Representative: Holliday, Louise Caroline
(86) International application number: PCT/JP2002/007879
(87) International publication number: WO 2003/014338

(57) **Abstract**

The present invention relates to an advantageous process for industrially producing an inactivated virus (e.g., HVJ, etc.) envelope. To solve the problem, the present invention provides a process for treating a virus with an alkylating agent to produce an inactivated virus envelope. A vector for introducing a biological macromolecule, such as a gene or the like, which is prepared from the inactivated HVJ envelope of the present invention, can be used for genetic function analysis or gene therapy. Specifically, the present invention comprises the steps of (a) inactivating a virus with an alkylating agent, (b) obtaining a condensate solution of the virus or the inactivated virus, and (c) purifying the virus or the inactivated virus by column chromatography and then ultrafiltration. The present invention also provides a composition and pharmaceutical agent which utilize an envelope obtained by the process of the present invention.

## Description

### TECHNICAL FIELD

The present invention relates to an industrial production process for inactivating virus (hereinafter, for example, Sendai virus (also, referred to as HVJ)) to obtain an inactivated virus envelope. The inactivated virus envelope is used as a reagent for use in preparing a vector capable of introducing a biological macromolecule, such as a gene, or the like.

### BACKGROUND ART

A number of virus methods and non-virus methods have been developed to introduce genes into cultured cells or biological tissues for the purposes of gene function analysis, gene therapy, and the like (Mulligan, Science, 260, 926 to 932, 1993; and Ledley, Human Gene Therapy, Vol. 6, 1129 to 1144, 1995). Virus methods are more effective for delivery of genes into cells. However, virus vectors may raise problems due to co-introduction of gene elements essential for parent genes derived from the parent virus, expression of virus genes, immunogenicity, or the like. On the other hand, a liposome method, which is a non-virus method, has a lower level of cytotoxicity and immunogenicity than that of virus methods, but tends to have a lower level of gene introduction efficiency into biological tissues that that of virus vectors.

HVJ was first reported as fusing Ehrlich tumor cells (Okada, Biken Journal, 1, 103-110, 1958), then the clarification of the mechanism of the ability to fuse cell membranes (hereinafter referred to as "fusion activity") has proceeded and meanwhile the use of it as a gene introduction vector has been studied. It is known that HVJ has a high level of immunogenicity, and particularly induces CTL when a large amount of NP protein is produced (Cole G.A. et al., Journal of Immunology, 158, 4301 to 4309, 1997). It is also likely that HVJ inhibits protein synthesis in hosts. Toavoid these problems, a technique was devised, in which a liposome including a gene or protein is fused with HVJ which has been inactivated by ultraviolet irradiation to prepare a fusion particule (HVJ-liposome). This technique made it possible to introduce a gene non-invasively into cultured cells or organisms (US Patent No. 5,631,237; Dzau et al., Proc. Natl. Acad. Sci. USA, 93, 11421 to 11425, 1996; and Kaneda et al., Molecular Medicine Today, 5, 298 to 303, 1999). However, the technique requires preparation of two different vehicles, a liposome and a viral envelope, which makes the technique complicated. The fusion particle of a liposome and HVJ disadvantageously has an average diameter about 1.3 times that of HVJ and a fusion activity one-tenth that of HVJ. In addition, for conventional HVJ-based vectors, there are some tissues in which it is not possible to introduce genes, or if it is possible, it is only possible with very low efficiency.

The present inventors have provided various novel inactivated virus envelope vectors for introducing a gene or oligonucleotide into cultured cells or organisms (International Application PCT/JP01/00782). Specifically, by packaging genes into various viruses (e.g., HVJ, etc.) whose genome is previously inactivated, the resultant viruses can be used as vectors capable of introducing genes into cultured cells or biological tissues with simplicity and high efficiency. These viral vectors are also less toxic to cells. According to the above-described background, there is a demand for an industrial production process of inactivated virus envelopes, which is inexpensive and effective, and can secure constant quality.

### (Problems to be Solved by the Invention)

For inactivated virus (e.g., HVJ, etc.) envelope vectors, the vectors need to be highly purified and retain a sufficient level of fusion activity for their purposes. Mass production of virus (e.g., HVJ, etc.) particles always has difficulty in achieving both purification and retainment of fusion activity. Also, the fusion activity of viruses needs to be retained after inactivation.

There are known methods for inactivating viruses for various vaccine production processes. However, it is difficult to use these methods without modification to inactivate viruses for envelope vectors. Vaccine production only requires the retainment of viral antigenicity, but not necessarily the non-impaired fusion activity of envelope viruses which is crucial to vector vehicles. The above-mentioned US Patent No. 5,631,237 discloses the inactivation of HVJ by ultraviolet irradiation. This technique has difficulty in the control of the level of irradiation and the prevention of nonuniform irradiation. Therefore, the technique is not suitable for the mass production of inactivated viruses (e.g., HVJ, etc.), i.e., inactivated virus (e.g., HVJ, etc.) envelopes. Therefore, an object of the present invention is to provide an industrial production process of inactivated virus (e.g., HVJ, etc.) envelopes.

The present inventors have diligently made attempts to establish an industrial production process of inactivated virus (e.g., HVJ, etc.) envelopes. As a result, the present invention was completed.

### DISCLOSURE OF THE INVENTION

The present invention relates to a process for producing an inactivated virus (e.g., HVJ, etc.) envelope, which is characterized by inactivating a virus (e.g., HVJ, etc.) with an alkylating agent. In another aspect of the present invention, a process for producing an inactivated virus (e.g., HVJ, etc.) envelope is provided, which comprises the steps of: (a) Inactivating a virus (e.g., HVJ, etc.) with an alkylating agent; (b) obtaining a condensate solution of the virus or the inactivated virus; and (c) purifying the virus or the inactivated virus by column chromatography and then ultrafiltration. The order of these steps may be rearranged as appropriate.

Therefore, the present invention is provided below by way of various embodiments of the present invention.
1. A process for producing an inactivated virus envelope, comprising the step of:
   inactivating a virus with an alkylating agent.
2. A process for producing an inactivated virus envelope, comprising the steps of:
   (a) inactivating a virus with an alkylating agent;
   (b) obtaining a condensate solution of the virus or the inactivated virus; and
   (c) purifying the virus or the inactivated virus by column chromatography and then ultrafiltration.
3. A process according to item 2, comprising the steps of:
   (a) inactivating a virus with an alkylating agent;
   (b) obtaining a condensate solution of the virus or the inactivated virus; and
   (c) purifying the virus or the inactivated virus by column chromatography and then ultrafiltration,
   wherein the steps are conducted in this order.
4. A process according to item 2, comprising the steps of:
   (b) obtaining a condensate solution of a virus;
   (a) inactivating the condensed virus with an alkylating agent; and
   (c) purifying the inactivated virus by column chromatography and then ultrafiltration,
   wherein the steps are conducted in this order.
5. A process according to item 2, comprising the steps of:
   (c) purifying a virus by column chromatography and then ultrafiltration;
   (a) inactivating the purified virus with an alkylating agent; and
   (b) obtaining a condensate solution of the inactivated virus,
   wherein the steps are conducted in this order.
6. A process according to any one of items 2, 3, 4, and 5, wherein the step of obtaining the condensate solution of the virus or the step of obtaining the condensate solution of the inactivated virus comprises performing centrifugation.
7. A process according to any one of items 2, 3, 4, and 5, wherein the step of obtaining the condensate solution of the virus or the step of obtaining the condensate solution of the inactivated virus comprises performing ultrafiltration.
8. A process according to item 1 or 2, wherein the virus is Sendai virus or influenza virus.
9. A process according to item 1 or 2, wherein the virus is Sendai virus.
10. A process for producing a composition comprising a viral envelope, comprising the steps of:
   (a) inactivating a virus with an alkylating agent;
   (b) obtaining a condensate solution of the virus or the inactivated virus; and
   (c) purifying the virus or the inactivated virus by column chromatography and then ultrafiltration;
      wherein the steps are conducted in any order, and subsequently,
   (d) mixing the purified inactivated virus with a material to be introduced therewith.
11. A process according to item 10, wherein the material is a biological macromolecule.
12. A process according to item 10, wherein the material is selected from the group consisting of nucleic acids, polypeptides, sugars, lipids, and complexes thereof.
13. A process according to item 10, wherein the virus is Sendai virus or influenza virus.
14. A process according to item 10, wherein the virus is Sendai virus.
15. A process for producing a medicament comprising a viral envelope, comprising the steps of:
   (a) inactivating a virus with an alkylating agent;
   (b) obtaining a condensate solution of the virus or the inactivated virus; and
   (c) purifying the virus or the inactivated virus by column chromatography and then ultrafiltration;
      wherein the steps are conducted in any order, and subsequently,
   (d) mixing the purified inactivated virus with a medical ingredient to be introduced therewith.
16. A process according to item 15, wherein the medical ingredient is a biological macromolecule.
17. A process according to item 15, wherein the medical ingredient is selected from the group consisting of nucleic acids, polypeptides, sugars, lipids, and complexes thereof.
18. A process according to item 15, wherein the medical ingredient is a nucleic acid encoding a polypeptide expressed in a host to which the nucleic acid is introduced.
19. A process according to item 15, wherein the medical ingredient is in the form of a vaccine.
20. A process according to item 15, wherein the virus is Sendai virus or influenza virus.
21. A process according to item 15, wherein the virus is Sendai virus.
22. A composition obtained by a process according to any one of items 10 to 14.
23. A medicament obtained by a process according to any one of items 15 to 21.

### BEST MODE FOR CARRYING OUT THE INVENTION

It should be understood throughout the present specification that articles for a singular form (e.g. , "a", "an", "the", etc. in English; "ein", "der", "das", "die", etc. and their inflections in German; "un", "une", "le", "la", etc. in French; "un", "una", "el", "la", etc. in Spanish, and articles, adjectives, etc. in other languages) include the concept of their plurality unless otherwise mentioned. It should be also understood that the terms as used herein have definitions typically used in the art unless otherwise mentioned.

Terms specifically used herein will be described below.

As used herein, the term "virus" refers to a transmissible small structure which has DNA or RNA as its genome and proliferates only within infected cells. Viruses include a virus belonging to a family selected from the group consisting of the family Retroviridae, the family Togaviridae, the family Coronaviridae, the family Flaviviridae, the family Paramyxoviridae, the family Orthomyxoviridae, the family Bunyaviridae, the family Rhabdoviridae, the family Poxviridae, the family Herpesviridae, the family Baculoviridarie, and the family Hepadnaviridae. A virus used herein may be preferably influenza virus or Sendai virus of the family Orthomyxoviridae. More preferably, a virus used herein is Sendai virus.

As used herein, the term "Sendai virus" or "HVJ" (Hemagglutinating virus of Japan) are used interchangeably, referring to a virus capable of cell fusion of the genus paramyxovirus of the family paramyxovirus. M. Kuroya et al. reported Sendai virus (1953). The genome is a minus strand of RNA having a base length of about 15,500. Sendai virus is a virus particle having an envelope and having a diameter of 150 nm to 300 nm (polymorphism). Sendai virus has RNA polymerase. The virus is unstable to heat, and causes agglutination of substantially all types of red blood cells, and hemolysis. The virus grows in cytoplasm of developing chicken eggs and/or cultured cells derived from the kidney of various animals. When established cells are infected with Sendai virus, persistent infection is likely to occur. The virus has an ability to fuse various cells, and therefore, is widely used for cell fusion in formation of heterokaryons, preparation of hybrid cells, and the like.

As used herein, the term " (virus or viral) envelope" refers to a membrane structure which basically comprises a lipid bilayer surrounding a nucleocapsid which exists in specific viruses, such as Sendai virus and the like. Envelopes are typically observed in mature viruses budding from cells. An envelope generally consists of host-derived lipids and small projecting structures consisting of spike proteins encoded by viral genes.

As used herein, the term "alkylation" refers to an action which substitutes an alkyl group for a hydrogen atom of an organic compound. The term "alkylating agent" refers to a compound which supplies an alkyl group. Examples of alkylating agents include, but are not limited to organic metal compounds, such as alkyl halide, dialkyl sulfate, alkyl sulfonate, alkyl lead, and the like. Examples of preferable alkylating agents include, but are not limited to, β-proplolactone, butyrolactone, methyl iodide, ethyl iodide, propyl iodide, methyl bromide, ethyl bromide, propyl bromide, dimethyl sulfate, diethyl sulfate, and the like.

As used herein, the term "inactivation" in relation to a virus (e.g., Sendai virus, etc.) indicates that the genome of the virus is inactivated. The inactivated virus is incapable of replication. Inactivation is achieved by a method described herein.

As used herein, the term "column chromatography" refers to liquid chromatography which uses a column filled with insoluble solid phase materials. By selecting a solid phase material and a mobile phase as appropriate, it is possible to separate and elute solutes based on the size, polarity, charge, or the like of molecules or ions. Examples of column chromatography include, but are limited to, anion exchange chromatography, cation exchange chromatography, size exclusion chromatography, affinity chromatography, hydrophobic interaction chromatography, gel filtration chromatography, and the like. Preferable column chromatography may be anion exchange chromatography.

As used herein, the term "ultrafiltration" refers to filtration at the molecular level, which may be used to separate large solute molecules from small solute molecules, or solute molecules from solvent molecules, and the like. Examples of ultrafiltration include, but are not limited to, gel filtration, semipermeable membrane filtration, and the like. Preferably, ultrafiltration may be, but is limited to, tangential ultrafiltration.

As used herein, the term "biological activity" refers to the activity which a certain factor (e.g., virus, polynucleotide or polypeptide) has within an organism, including activity exhibiting various functions. For example, when the certain factor is a transcriptional factor, its biological activity includes activity to regulate transcriptional activity. When the certain factor is a virus, its biological activity includes infection activity. As another example, when the certain factor is a ligand, its biological activity includes binding to a receptor to which the ligand corresponds.

As used herein, "nucleic acid", "nucleic acid molecule", "polynucleotide", and "oligonucleotide" are herein used interchangeably to refer to macromolecules (polymer) comprising a series of nucleotides, unless otherwise specified. A nucleotide refers to a nucleoside whose base is a phosphoric ester. The base of the nucleotide is a pyrimidine or purine base (pyrimidine nucleotide and purine nucleotide). Polynucleotides include DNA or RNA.

As used herein, "nucleotide" refers to any naturally occurring nucleotide and non-naturally occurring nucleotide. "Derived nucleotide" refers to a nucleotide which is different from naturally occurring nucleotides but has a function similar to that of its original naturally occurring nucleotide. Such derived nucleotides are well known in the art.

As used herein, the term "fragment" in relation to a nucleic acid molecule refers to a polynucleotide which has a length which is smaller than the full length of the reference nucleic acid molecule and is sufficient as an agent of the present invention. Therefore, the term "fragment" refers to a polynucleotide which has a sequence length ranging from 1 to n-1 with respect to the full length of the reference polynucleotide (of length n). The length of the fragment can be appropriately changed depending on the purpose. For example, the lower limit of the length of the fragment includes 5, 6, 7, 8, 9, 10, 15, 20, 25, 30, 40, 50, 75, 100 or more nucleotides. Lengths represented by integers which are not herein specified (e.g., 11 and the like) may be appropriate as a lower limit. Homology may be represented by a score measured by a search program BLAST using an algorithm denveloped by Altschul et al. (J. Mol. Biol., 215, 403-410 (1990)).

As used herein, the terms "protein", "polypeptide", and "peptide" are used interchangeably, referring to a macromolecule which consists of a series of amino acids. The term "amino acid" refers to an organic molecule which has a carboxyl group and an amino group bound to a carbon atom. Preferably, amino acids herein include, but are not limited to, 20 naturally-occurring amino acids.

As used herein, the term "gene" refers to an element defining a genetic trait. A gene is typically arranged in a given sequence on a chromosome. A gene which defines the primary structure of a protein is called a structural gene. A gene which regulates the expression of a structural gene is called a regulatory gene. As used herein, the term "gene" may refer to "polynucleotide", "oligonucleotide", "nucleic acid", and "nucleic acid molecule" and/or "protein", "polypeptide", "oligopeptide" and "peptide".

As used herein, the term "expression" of a gene product, such as a gene, a polynucleotide, a polypeptide, or the like, indicates that the gene or the like is affected by a predetermined action *in vivo* to be changed into another form. Preferably, the term "expression" indicates that genes, polynucleotides, or the like are transcribed and translated into polypeptides. In one embodiment of the present invention, genes may be transcribed into mRNA. More preferably, these polypeptides may have post-translational processing modifications. As used herein, the term "regulation" in relation to the expression of a gene refers to, but is not limited to, enhancement, reduction, induction, elimination, deceleration, acceleration, and the like of gene expression.

Examples of a gene to be treated include, but are not limited to genes encoding enzymes, hormones, lymphokines, receptors, growth factors, regulatory proteins, polypeptides affecting the immune system, immunoregulatory factors, antibodies, and the like. Specifically, these genes include, but are not limited to, genes encoding human growth hormones, insulin, interleukin-2, tumor necrosis factors, nerve growth factors (NGFs), epithelial growth factors, tissue plasminogen activators (TPAs), Factor VIII:C, calcitonin, thymidine kinase, interferon, granulocyte-macrophage colony-stimulating factors (GMCSFs), erythropoietin (EPO), hepatocyte growth factors (HGFs), and the like. These genes may be present in the form of a nucleic acid or a polypeptide in a medicament of the present invention.

Examples of a vaccine which may be herein used as a medicament include, but are not limited to, vaccines for cancer, acquired immunodeficiency syndrome, measles, herpes simplex, and the like. These vaccines may be present in the form of a nucleic acid or a polypeptide in a medicament of the present invention.

The present invention provides a pharmaceutical composition or a medicament comprising the above-described envelope singly or in combination with a stabilizing compound, a diluent, a carrier, other ingredients, or other pharmaceutical agents. Preferably, the present invention may be in the form of a vaccine or in other forms suitable for gene therapy.

A pharmaceutical composition and medicament of the present invention may be used in a form which allows the envelope thereof to be taken into cells at an affected site or cells of a tissue of interest.

A pharmaceutical composition and medicament of the present invention may be administered within any aseptic biocompatible pharmaceutical carrier including, but not being limited to, physiological saline, buffered physiological saline, dextrose, water, and the like. Any of these molecules may be administered into patients within a pharmaceutical composition, which is mixed with an appropriate excipient, adjuvant, and/or pharmaceutically acceptable carrier, singly or in combination with other pharmaceutical agents. In a certain embodiment of the present invention, a pharmaceutically acceptable carrier is pharmaceutically inactive.

A pharmaceutical composition and medicament of the present invention is administered orally or parenterally. Examples of parenteral delivery methods include, but are not limited to, topical, intraarterial (e.g., via the carotid artery, or the like), intramusclar, subcutaneous, intramedullary, subarachnoideal, intraventicular, intravenous, intraperitoneal, and intranasal administrations, and the like. In the present invention, any route which allows delivery to a site to be treated may be used.

In addition to an envelope, these pharmaceutical compositions and pharmaceutical agents may comprise a pharmaceutically acceptable carrier containing other compounds for promoting processing of the envelope in order to prepare an excipient or pharmaceutically acceptable preparation. Further details of prescription and administration are described in, for example, the latest edition of Japanese Pharmacopeia and its latest supplement, the latest edition of "REMINGTON'S PHARMACEUTICAL SCIENCES" (Maack Publishing Co., Easton, PA), or the like.

A pharmaceutical composition for oral administration may be prepared using a pharmaceutically acceptable carrier well known in the art in an administration form suitable for administration. Such a carrier can be prepared as a tablet, a pill, a sugar-coated agent, a capsule, a liquid, a gel, a syrup, a slurry, a suspension, or the like, which is suited for the patient to take the pharmaceutical composition.

The pharmaceutical composition for oral use may be obtained in the following manner: an active compound is combined with a solid excipient, the resultant mixture is pulverized if necessary, an appropriate compound is further added if necessary to obtain a tablet or the core of a sugar-coated agent, and the granular mixture is processed. The appropriate excipient may be a carbohydrate or protein filler, including, but not being limited to, the following: sugar including lactose, sucrose, mannitol, or sorbitol; starch derived from maize, wheat, rice, potato, or other plants; cellulose such as methylcellulose, hydroxypropylmethylcellulose, or sodium carboxymethylcellulose; and gum including gum Arabic and gum tragacanth; and proteins such as gelatin and collagen. A disintegrant or a solubilizing agent such as crosslinked polyvinyl pyrrolidone, agar, alginic acid or a salt thereof (e.g., sodium alginate) may be used if necessary.

The sugar-coated agent core is provided along with an appropriate coating, such as a condensed sugar solution. The sugar-coated agent core may also contain gum arabic, talc, polyvinyl pyrrolidone, carbopolygel, polyethylene glycol, and/or titanium dioxide, a lacquer solution, and an appropriate organic solvent or a mixed solvent solution. To identify a product, or characterize the amount of an active compound (i.e., dose), dye or pigment may be added to tablets or sugar-coated agents.

The pharmaceutical preparation which may be orally used may contain, for example, a soft sealed capsule consisting of a gelatin capsule, gelatin and coating (e.g., glycerol or sorbitol). The gelatin capsule may contain an active ingredient mixed with a filler or binder such as lactose or starch, a lubricant such as talc or magnesium stearate, and optionally a stabilizer. In the soft capsule, the decoy compound may be dissolved or suspended in an appropriate liquid, such as fatty oil, liquid paraffin or liquid polyethylene glycol, with or without a stabilizer.

The pharmaceutical preparation for parenteral administration contains an aqueous solution of an active compound. For the purpose of injection, the pharmaceutical composition of the present invention is prepared in an aqueous solution, preferably Hank's solution, Ringer's solution, or a physiologically suitable buffer such as a buffered physiological saline. The aqueous suspension for injection may contain a substance for increasing the viscosity of a suspension (e.g., sodium carboxymethylcellulose, sorbitol, or dextran). Further, the suspension of the active compound may be prepared as an appropriate oily suspension. Appropriate lipophilic solvents or vehicles include fatty acid such as sesame oil, synthetic fatty acid esters such as ethyl oleate or triglycerides, or liposomes. The suspension may contain a stabilizer which allows a high-concentration solution preparation, or an appropriate pharmaceutical agent or reagent for increasing the solubility of the compound, if necessary.

The pharmaceutical composition of the present invention may be produced using a process similar to processes known in the art (e.g., conventional mixing, dissolution, rendering to granules, preparation of a sugar-coated agent, elutriation, emulsification, capsulation, inclusion, or freeze drying).

A pharmaceutical composition of the present invention includes a composition containing an effective amount of an envelope of the present invention which can achieve the intended purpose of the decoy compound. "Therapeutically effective amount" and "pharmacologically effective amount" are terms which are well recognized by those skilled in the art and which refer to an amount of pharmaceutical agent effective for production of an intended pharmacological effect. Therefore, the therapeutically effective amount is an amount sufficient for reducing the manifestation of the disease to be treated. A useful assay for confirming an effective amount (e.g., a therapeutically effective amount) for a predetermined application is to measure the degree of recovery from a target disease. An amount actually administered depends on an Individual to be treated. The amount is preferably optimized so as to achieve a desired effect without a significant side effect. The determination of the therapeutically effective dose is within the ability of those skilled in the art.

A therapeutically effective dose of any compound can be initially estimated using either a cell culture assay or any appropriate animal model. The animal model is used to achieve a desired concentration range and an administration route. Thereafter, such information can be used to determine a dose and route useful for administration into humans.

The term "therapeutically effective amount" in relation to an envelope refers to an amount which results in amelioration of symptoms or conditions of a disease. The therapeutic effect and toxicity of an envelope may be determined by standard pharmaceutical procedures in cell cultures or experimental animals (e.g., ED₅₀, a dose therapeutically effective for 50% of a population; and LD₅₀, a dose lethal to 50% of a population). The dose ratio between therapeutic and toxic effects is the therapeutic index, and it can be expressed as the ratio of ED₅₀/LD₅₀. Pharmaceutical compositions which exhibit high therapeutic indices are preferable. The data obtained from cell culture assays and animal studies can be used in formulating a dosage range for use in humans. The dosage of such compounds lies preferably within a range of circulating concentrations that include the ED₅₀ but have little or no toxicity. Such a dosage may vary within this range depending upon the dosage form employed, the susceptibility of the patient, and the route of administration. As an example, the dose of an envelope is appropriately selected depending on the age and other conditions of a patient, the type of a disease, the type of the envelope employed, and the like.

When an envelope vector of the present invention is administered into a human, from 400 HAU to 400,000 HAU of the envelope vector may be administered per subject, preferably 1,200 HAU to 120,000 HAU, and more preferably 4,000 HAU to 40,000 HAU. The amount of an exogenous gene contained in an envelope to be administered may be from 2 µg to 2,000 µg per subject, preferably from 6 µg to 600 µg per subject, and more preferably from 20 µg to 200 µg.

As used herein, the term "HAU" refers to an amount of viral activity capable of agglutinating 0.5% of chicken red blood cells. 1 HAU corresponds to 24,000,000 virus particles (Okada Y. et al. , Biken Journal, 4, 209-213, 1961). The above-described amount can be administered, for example, from once per day to several times per day.

The exact dose is chosen by an individual physician in view of the condition of a patient to be treated. Doses and times of administration are adjusted to provide a sufficient level of the active portion, or to retain a desired effect. Additional factors to be considered include the severity of the condition of a disease (e.g., the size and location of a tumor; the age, weight and sex of a patient; diet-limiting time and frequency of administration, combination of drugs, susceptibility to reactions, and resistance/response to treatment). A sustained action pharmaceutical composition may be administered every 3 to 4 days, every week, or once per two weeks, depending on the half life and clearance rate of the specific preparation. Guidance for specific doses and delivery methods are provided in publications known in the art.

A composition and medicament the present invention may also comprise a biocompatible material. The biocompatible material may comprise at least one selected from the group consisting of silicone, collagen, gelatin, glycolic acid/lactic acid copolymer, ethylene/vinyl acetate copolymer, polyurethane, polyethylene, polytetrafluoroethylene, polypropylene, polyacrylate, and polymethacrylate. Silicone is preferable because it is easy to mold. Examples of biodegradable macromolecules include, but are not limited to, polymers, copolymers or mixtures thereof, which are synthesized by noncatalyzed hydration of at least one selected from the group consisting of collagen, gelatin, α-hydroxycarboxylic acids (e.g., glycolic acid, lactic acid, hydroxybutyric acid, etc.), hydroxydicarboxylic acids (e.g., malic acid, etc.), and hydroxytricarboxylic acids (e.g., citric acid, etc.); polyacid anhydrides (e.g., poly-α-cyanoacrylic ester, polyamino acid (e.g., poly-γ-benzyl-L-glutamic acid, etc.); maleic anhydride-based copolymers (e.g., styrene/maleic acid copolymer, etc.); and the like. The manner of polymerization may be any of random, block, and graft polmerization. When α-hydroxycarboxylic acids, hydroxydicarboxylic acids, or hydroxytricarboxylic acids have an optically active center within a molecule, any of D-isomers, L-isomers, and DL-isomers can be used. Preferably, glycolic acid/lactic acid copolymer may be used.

In one embodiment, a composition and medicament of the present invention may be provided in a sustained-release form. Any sustained-released dosage form may be used in the present invention. Examples of sustained-release dosage forms include, but are not limited to, rod-like formulations (e.g., pellet-like, cylinder-like, needle-like formulations, etc.), tablet formulations, disk-like formulations, sphere-like formulations, sheet-like formulations, and the like. Methods for preparing sustained-release dosage forms are well known in the art, as described in, for example, the Japanese Pharmacopeia, the U.S. Pharmacopeia, Pharmacopeias of other countries, and the like. Examples of a method for producing sustained-release drugs include, but are not limited to, a method using disaggregation of a drug from a complex, a method for preparing an aqueous suspension of liquid drug, a method for preparing an oil injection solution or oil suspended injection solution, a method for preparing an emulsified injection solution (o/w or w/o type emulsified injection solution, or the like), and the like.

The use of the composition and medicament of the present invention is usually performed under the supervision of a doctor, or without supervision of a doctor if approved by an authority and a law of a country in which the present invention is used.

The present invention may also be administered preferably in the form of a vaccine. A vaccine means an antigen in any of various forms (e.g., protein, DNA, and the like) which is used to prevent (or treat) a certain type of disease (e.g., contagious diseases, infectious diseases, and the like). Attenuated live pathogens (live vaccine), inactive pathogens (or a part thereof), metabolites of a pathogen (toxin, inactivated toxin (i.e., toxoid), or the like), DNA vaccines, or the like are used depending on the type of infection, transmission, epidemic, or the like. Vaccination actively develops immunity (humoral immunity, cell-mediated immunity, or both) within the body of organisms (humans, livestock, and vectors) and prevents infection, transmission, epidemic, or the like caused by pathogens.

The vaccines of the present invention are not particularly limited to any dosage form, and are prepared in accordance with methods known in the art. Further, the vaccines of the present invention may be in the form of an emulsion containing various adjuvants. The adjuvants aid sustenance of a high level of immunity when the above-described HSV gene recombinant is used in a smaller dose than when it is used alone. Examples of the adjuvants include Freund's adjuvant (complete or incomplete), adjuvant 65 (including peanut oil, mannide monooleate and aluminum monostearate), and aluminum hydrate, aluminum phosphate or mineral gel such as alum. For vaccines for humans or animals used as a food source, adjuvant 65 is preferable. For vaccines for commercial animals, mineral gel is preferable.

In addition to the above-described adjuvants, the vaccines of the present invention may contain at least one additive for preparations selected from diluents, aroma chemicals, preservatives, excipients, disintegrants, lubricants, binders, surfactants, plasticizers, and the like.

The administration routes of the vaccines of the present invention are not particularly limited. However, the vaccines are preferably administered parenterally (e.g., intravenously, intraarterially, subcutaneously, intradermal, intramuscularly or intraperitoneally).

The dose of the vaccines of the present invention can be selected depending on various conditions: what administration is intended; whether infection is primary or recurrent; the age and weight, conditions of patients; the severity of disease; and the like. When intended to treat diseases caused by recurrent infection, the dose of the vaccines of the present invention is preferably about 0.01 ng to 10 mg per kg weight, and more preferably about 0.1 ng to 1 mg.

The number of administrations of the vaccines of the present invention varies depending on the above-described various conditions, and is not necessarily determined in the same manner. However, preferably, the vaccines are repeatedly administered at the intervals of days or weeks. Particularly, administration is conducted at a total of several times, or preferably about one to two times, at the interval of about 2 to 4 weeks. The number of administrations (administration time) is preferably determined by symptomatology or a fundamental test using antibody titer while monitoring the conditions of diseases.

Compositions (e.g., vaccines) are herein provided for treating or preventing pathogen infections (e.g., viruses (e.g., HIV, influenza virus, rotavirus, and the like), or bacteria). Such compositions comprise at least one gene or protein of the pathogen. The exogenous gene preferably is full length but may be a partial sequence as long as it contains at least an epitope capable of triggering immunity. The term "epitope" as used herein refers to an antigenic determinant whose structure has been revealed. A method for determining an epitope is known in the art. Once the primary nucleic acid or amino acid sequence of a protein is provided, such epitopes can be determined by such a known routine technique. A useful epitope may have at least a length of three amino acids, preferably, at least 4 amino acids, at least 5 amino acids, at least 6 amino acids, at least 7 amino acids, at least 8 amino acids, at least 9 amino acids, at least 10 amino acids, at least 15 amino acids, at least 20 amino acids, or at least 25 amino acids.

As used herein, the term "neutralizing antibody" refers to an antibody which is involved in a neutralizing reaction which neutralizes the biological activity of an antigen, such as an enzyme, a toxin, a bacterium, a virus, or the like. The term "neutralizing reaction" refers to a reaction in which an antigen is bound to a neutralizing antibody, so that the activity of the antigen and the antibody is eliminated or lowered. If a vaccine is administered, a neutralizing antibody is produced and serves to get rid of pathogens.

As used herein, the term "gene therapy" or "gene therapeutic method" refers to a method for treating diseases caused by a damaged (or defective) gene by introducing a healthy or modified nucleic acid (e.g., DNA) to patients. Some gene therapies use the step of injecting a nucleic acid without any protection or cover, though any vectors are often used. An envelope of the present invention may be used as such a vector.

In another aspect, the present invention provides a kit comprising a composition and medicament. The kit comprises a composition and medicament of the present invention; and instructions which provide guidance in administering the composition and medicament. The instructions describe a statement indicating an appropriate method for administering a composition or a medicament of the present invention. The instructions are prepared in accordance with a format defined by an authority of a country in which the present invention is practiced (e.g., Health, Labor and Welfare Ministry in Japan, Food and Drug Administration (FDA) in the U.S., and the like), explicitly describing that the instructions are approved by the authority. The instructions are so-called package insert and are typically provided in paper media. The instructions are not so limited and may be provided in the form of electronic media (e.g., web sites, electronic mails, and the like provided on the Internet).

The amount of a composition and medicament used in the process of the present invention can be easily determined by those skilled in the art with reference to the purpose of use, a target disease (type, severity, and the like), the patient's age, weight, sex, and case history, the form or type of the cell physiologically active substance, and the like.

The frequency of the treatment method of the present invention applied to a sub j ect (or patient) is also determined by those skilled in the art with respect to the purpose of use, target disease (type, severity, and the like), the patient's age, weight, sex, and case history, the progression of the therapy, and the like. Examples of the frequency include once per day to several months (e.g., once per week to once per month). Preferably, administration is performed once per week to month with reference to the progression.

A composition and medicament of the present invention comprises a material or medical ingredient to be introduced into hosts. Such a material or medical ingredient may be a biological macromolecule. Preferably, such a biological macromolecule is selected from the group consisting of nucleic acids, polypeptides, sugars, lipids, and complexes thereof. Preferably, such a medical ingredient may be a nucleic acid encoding a polypeptide which is expressed in the host into which the ingredient is introduced.

A composition and medicament of the present invention may comprise one or more additional medical ingredients. Such a medical ingredient may be contained in the pharmaceutical composition. Examples of such a medical ingredient include, but are not limited to, those described below:
central nerve system drugs (e.g., general anesthetics, sedative-hypnotics, anxiolytics, antiepileptics, anti-inflammatory agents, stimulants, antihypnotics, antiparkinson agents, antipsychotics, combination cold remedies, and the like);
peripheral nerve agents (e.g., local anesthetics, skeletal muscle relaxants, autonomic nerve agents, antispasmodic agents, and the like);
sensory organ drugs (e.g., ophthalmological agents, otorhinolaryngological agents, antidinics, and the like);
circulatory organ drugs (e.g., cardiotonics, antiarrhythmics, diuretics, antihypertensive agents, vasoconstrictors, vasodilators, antihyperlipemia agents, and the like);
respiratory organ drugs (e.g., respiratory stimulants, antitussives, expectorants, antitussive expectorants, bronchodilators, collutoriums, and the like);
digestive organ drugs (e.g., stegnotics, antiflatuents, peptic ulcer agents, stomachics, antacids, cathartics, enemas, cholagogues, and the like);
hormone agents (e.g., pituitary gland hormone agents, salivary gland hormone agents, thyroid gland hormone agents, accessory thyroid gland hormone agents, anabolic steroid agents, adrenal gland hormone agents, androgenic hormone agents, estrogen agents, progesterone agents, mixed hormone agents, and the like);
urogenital organ and anal drugs (e.g., urinary organ agents, genital organs agents, uterotonics, hemorrhoid agents, and the like);
dermatologic drugs (e.g., dermatologic disinfectants, wound protecting agents, pyogenic disease agents, analgesics, antipruritics, astringents, antiphlogistics, parasitic skin disease agents, emollients, hair agents, and the like);
dental and oral agents;
drugs for other organs;
vitamin agents (e.g., vitamin A agents, vitamin D agents, vitamin B agents, vitamin C agents, vitamin E agents, vitamin K agents, mixed vitamin agents, and the like);
nutritive agents (e.g., calcium agents, inorganic preparations, saccharide agents, protein amino acid preparations, organ preparations, infant preparations, and the like);
blood and body fluid drugs (e.g., blood substitute agents, styptics, anticoagulants, and the like);
dialysis drugs (e.g., kidney dialysis agents, peritoneal dialysis agents, and the like);
other metabolic drugs (e.g., organ disease agents, antidotes, antabuses, arthrifuges, enzyme preparations, diabetic agents, and others);
cell activating agents (e.g., chlorophyll preparations, pigment agents, and the like);
tumor agents (e.g., alkylation agents, antimetabolites, antineoplastic antibiotic preparations, antineoplastic plant extract preparations, and the like);
radiopharmaceuticals;
allergy drugs (e.g., antihistamic agents, irritation therapy agents, non-specific immunogen preparations, and other allergy drugs, crude drugs and drugs based on Chinese medicine, crude drugs, Chinese medicine preparations, and other preparations based on crude drug and Chinese medicine formulation);
antibiotic preparations (e.g., acting on gram-positive bacteria, gram-negative bacteria, gram-positive mycoplasmas, gram-negative mycoplasmas, gram-positive rickettsia, gram-negative rickettsia, acid-fast bacteria, molds, and the like);
chemotherapeutic agents (e.g., sulfa drugs, antitubercular agents, synthetic antimicrobial agents, antiviral agents, and the like);
biological preparations (e.g., vaccines, toxoids, antitoxins, leptospire antisera, blood preparations, biological test preparations, and other biological preparations, and antiprotozoal drugs, anthelmintics, and the like);
dispensing agents (e.g., excipients, ointment bases, solvents, flavors, colorants, and the like);
diagnostic drugs (e.g., contrast media, function testing reagents, and the like);
sanitation drugs (e.g., preservative);
xenodiagnostic drugs (e.g., cytologic examination drugs, and the like);
non-categorized drugs which do not aim mainly for therapy; and
narcotics (e.g., opium alkaloid drugs, coca alkaloid preparations, synthetic narcotics, and the like).

A composition and medicament of the present invention may be used for a human and may be used for other hosts.

Therefore, when a composition of the present invention is used as an agricultural chemical, the composition may concurrently comprise an active ingredient of agricultural chemicals as described below:
(herbicides) pyrazonate, daimuron, bromobutide, mefenacet, MCP, MCPB, triclopyr, naproanilide, CNP, chlomethoxynil, bifenox, MCC, pyributicarb, DCPA, napropamide, diphenamid, propyzamide, asulam, DCMU, linuron, methyldymron, tebuthiuron, bensulfuronmethyl, simazine, atrazine, simetryn, ametryn, prometryn, dimethametryn, metribuzin, bentazone, oxadiazon, pyrazonate, benzofenap, glyphosate, bilanafos, alloxydim, imazosulfuron, azimsulfuron, pyrazosulfuron, cinosulfuron;
(insecticides/acaricides) diazinon, fenthion, isoxathion, pyridaphenthion, fenitrothion, dimethoate, PMP, dimethylvinphos, acephate, DEP, NAC, MTMC, MIPC, PHC, MPMC, XMC, BPMC, bendiocarb, pirimicarb, methomyl, oxamyl, thiodicarb, cypermethrin, cartap hydrochloride, thiocyclam, bensultap, pyriproxyfen, phenoxycarb, methoprene, diflubenzuron, teflubenzuron, chlorfluazuron, buprofezin, hexythiazox, pyridaben, clofentezine, nitenpyram;
(bactericides) probenazole, isoprothiolane, pyroquilon, flutolanil, metominostrobin, ziram, thiram, captan, TPN, phthalide, tolclofos-methyl, fosetyl, thiophanate methyl, benomyl, carbendazole, thiabendazole, diethofencarb, iprodione, vinclozolin, procymidone, fluoroimide, oxycarboxin, mepronil, flutolanil, pencycuron, metalaxyl, oxadixyl, triadimefon, hexaconazole, triforine, blasticidin-S, kasugamycin, polyoxin, validamycin-A, mildiomycin, PCNB, hydroxyisoxazole, dazomet, dimethirimol, diclomezine, triazine, ferimzone, tricyclazole, oxolinic acid, and the like, and preferably strobilurin-based compounds, such as metominostrobin and the like.

When a composition of the present invention is used as an agricultural chemical, the composition may be mixed with an acaricide (e.g., chlorobenzilate, etc.), a plant growth regulator (e.g.,paclobutrazol, etc.), a nematocide (e.g., benomyl, etc.), a synergist (e.g., piperonyl butoxide, etc.), an attractant (e.g., eugenol, etc.), a repellent (e.g., creosote, etc.), a pigment (e.g., food blue No. 1, etc.), a fertilizer (e.g., urea, etc.), or the like.

Molecular biological techniques, biochemical techniques, and microbiological techniques used herein are well known and commonly used in the art, and are described in, for example, Ausubel F.A. et al. editors (1988), Current Protocols in Molecular Biology, Wiley, New York, NY; Sambrook J. et al. (1987), Molecular Cloning: A Laboratory Manual, 2nd Ed., Cold Spring Harbor Laboratory Press, Cold Spring Harbor, NY; Jikken Igaku [Experimental Medicine], "Experimental Methods for Gene Introduction & Expression Analysis", special issue, Yodo-sha, 1997; and the like.

Viruses (e.g., HVJ, etc.) are proliferated for use in the present invention as follows. A seed virus is inoculated into fertilized chicken eggs. Alternatively, a persistent infection line of cultured cells or tissue of a monkey or human is used (culture medium supplemented with a hydrolytic enzyme, such as trypsin or the like). Alternatively, cultured cells are infected with a cloned viral genome to elicit persistent infection. These mutant lines can be used in the present invention. In addition, viruses (e.g., HVJ, etc.), which can be obtained by other methods, can be used. Recombinant HVJ (Hasan M.K. et al., Journal of General Virology, 78, 2813 to 2830, 1997; or Yonemitsu Y. et al. , Nature Biotechnology, 18, 970 to 973, 2000) can be used. Any HVJ may be used. The Z line (e.g., Accession No. ATCC VA 2388 or one available from Charles River SPAFAS) or the Cantell line (e.g., Johnston M.D., J. Gen. Virol., 56, 175 to 184, 1981 or one available from Charles River SPAFAS) are more desirable.

(a) The step of inactivating HVJ with an alkylating agent comprises the following procedure. The alkylating agent is added to culture medium or chorioallantoic fluid containing HVJ or its condensate solution to a concentration of 0.004% to 0.010%, more desirably 0.006% to 0.008%, followed by incubation at 0°C to 25°C for 30 minutes to 2 hours, more desirably at room temperature for 1 hour. Thereafter, incubation is conducted for 1 to 3 hours while the temperature is kept at about 37°C. In this procedure, the alkylating agent itself is inactivated. HVJ can be preserved at low temperature for several days until the next step. In the inactivating step, by alkylating various structural components of a virus (e.g., a lipid, a protein, a nucleic acid, etc.), the proliferating ability of the virus is chemically inactivated, while the fusion activity of the viral envelope is retained.

In a preferred embodiment, β-propiolactone, anion exchange chromatography, and tangential ultrafiltration are used as an alkylating agent, a column chromatography method, and an ultrafiltration method, respectively, unless otherwise mentioned. The present invention is not limited to this. Therefore, other alkylating agents, other column chromatography methods, and other ultrafiltration methods may also be employed.

Inactivated HVJ is evaluated by determining the presence or absence of infection of HVJ in cultured cells. After HVJ is inactivated, cells of a monkey kidney cell line LLC-MK2 cell are infected with the inactivated HVJ at 37°C for 1 hour. The one-step growth of HVJ takes place 12 to 18 hours after infection. Therefore, the cells are incubated at 37°C in the presence of carbon dioxide gas for 18 hours to 24 hours. Thereafter, the cells are fixed with acetone/methanol. The presence or absence of HVJ expressed in the cells infected with HVJ is determined by immunological staining using antibodies against F protein. Specifically, HVJ is solubilized with a surfactant NP-40 (nonylphenoxypolyethoxyethanol), followed by centrifugation to isolate membrane components. The isolated membrane components are subjected to ion exchange chromatography to obtain F protein (Yoshima H. et al., J. Biol. Chem., 1981; and Suzuki K. et al., Gene Therapy and Regulation, 2000). Thereafter, the F protein is inoculated in conjunction with Freund's adjuvant into rabbits 4 times to obtain antiserum against F protein (anti-F protein polyclonal antibodies of rabbit (primary antibodies)). The fixed cells are treated with the primary antibodies for 1 hour, followed by treatment with anti-rabbit IgG polyclonal antibodies labeled with FITC of pig (secondary antibodies) for 1 hour. After treatment with the secondary antibodies, the cells are observed under a fluorescence microscope to evaluate the inactivation of HVJ.

The influence of the inactivating treatment on the membrane function of virus (e.g., HVJ, etc.) envelopes can be represented by the HA activity of the inactivated virus (e.g., HVJ, etc.) envelope. The HA activity can be measured by commonly used methods. A suspension of inactivated HVJ envelope is placed into 3 wells of a 96-well plate (round base) in an amount of 50 µl, 40µl, and 30µl, respectively. Thereafter, the suspension is serially diluted 2-fold with PBS(-) (Dulbecco' s Phosphate Buffer Saline free from Mg ions and Ca ions) to prepare serially diluted samples. Thereafter, 0.5% chicken red blood cell solution is added, followed by incubation at 2°C to 6°C for 2 hours. Thereafter, the presence or absence of an agglutination reaction is examined. The HA activity is calculated based on the amount of a sample in the sample series which loses an agglutination reaction and the inverse of the dilution factor of the well.

The influence of inactivating treatment on gene introduction efficiency can be evaluated by calculating the ratio of cells having an introduced gene to cells targeted by gene introduction or the total amount of genes introduced into targeted cells.

The above-described ratio can be evaluated by introducing a gene encoding a fluorescent protein (EGFP, Mosser D.D. et al., Biotechniques, 1997). Briefly, after inactivation, virus envelopes are treated with a surfactant and protamine sulfate to include an expression plasmid (e.g., available from Clontech) having a fluorescent protein (EGFP, Mosser D.D. et al., Biotechniques, 1997) gene. Thereafter, the virus envelope is introduced into cells of a hamster kidney cell line BHK-21. After introduction of EGFP expression plasmid, the cells are cultured at 37°C in the presence of carbon dioxide gas for 24 hours. Thereafter, the expression of EGFP is observed under a fluorescence microscope. The cells are suspended by trypsin/EDTA treatment. The presence or absence of EGFP expression and the expression level of EGFP are analyzed by flow cytometry (EPICS) to evaluate introduction efficiency.

The above-described total amount of genes can be evaluated by introducing a luciferase gene. Similar to the above-described method, an expression plasmid (pGL3) containing a luciferase gene is introduced into cells of the hamster kidney cell line BHK-21, followed by culture in the presence of carbon dioxide gas for 20 hours to 24 hours. Thereafter, the expression level of luciferase is measured using a measurement kit (LucLite, produced by Packard). The amount of light emission can be measured using LUMINOMETER (TD-20e, produced by Turner).

(b) In the step of obtaining a condensate solution of a virus (e.g., HVJ, etc.), cell culture medium or chorioallantoic fluid containing proliferated viruses (e.g., HVJ, etc.) can be subjected to ultrafiltration, centrifugation, or the like. Ultrafiltration can purify the virus (first stage) as well as condensation of the virus. The resultant buffered solution can be used in the subsequent step. In a preferred embodiment, ultrafiltration is conducted. For ultrafiltration, various systems, such as spiral membranes, flat membranes, hollow fibers, and the like, can be used. Preferably, the cut-off threshold may be smaller than the particle diameter of the virus. Other condensation methods may be used as appropriate.

Viruses are condensed by, for example, high-speed centrifugation, density gradient ultracentrifugation, or a combination thereof. In these centrifugation methods, in addition to condensation of viruses, a buffered solution of the preparation may be subjected to exchange in view of the subsequent step. High-speed centrifugation may be conducted at 15,000×g to 30,000×g. Density gradient ultracentrifugation may be conducted at 50,000×g to 100,000×g. In any case, centrifugation is desirably conducted at low temperature, particularly 2°C to 6°C, for example. High-speed centrifugation and ultracentrifugation may be combined from one to several times for each. Examples of density gradient ultracentrifugation include, but are not limited to, sucrose density gradient centrifugation, potassium bromide density gradient centrifugation, cesium chloride density gradient centrifugation, and the like. In the case of sucrose density gradient centrifugation, a virus suspension is placed on sucrose solution (30% to 60% w/v), followed by centrifugation (50,000×g to 100,000×g). A band on a sucrose solution layer is recovered. Large-amount sucrose density gradient centrifugation can be performed with high efficiency using a zonal rotor. The step of obtaining a condensate solution of an inactivated virus (e.g., HVJ, etc.) can be carried out by ultrafiltration, centrifugation, or the like as in the above-described step of obtaining a condensate solution of a virus (e.g., HVJ, etc.). Although, ultrafiltration is more desirable.

Prior to the above-described step of obtaining a condensate solution of a virus (e.g., HVJ, etc.) or an inactivated virus (e.g., HVJ, etc.) (by ultrafiltration or centrifugation), a pretreatment may be optionally performed so as to remove residues, such as tissue fragments or the like, from culture medium or chorioallantoic fluid containing the virus. The pretreatment may be performed by filtration or low-speed centrifugation (at 2000 rpm to 4000 rpm for 10 min to 20 min), for example. Filtration is desirable for a large amount of solution containing HVJ or inactivated HVJ. Filtration is performed using a membrane having a pore having a small diameter which is sufficient for an HVJ particle to pass therethrough and for residues to be retained. A deep filter having a gradually decreasing pore diameter, a flat membrane, or a hollow threadmay be used for precise filtration. More specifically, Polygard-CR, Lifegard, or Polygard-CN (all available from Millipore) may be used. A filter having a pore whose diameter is larger than the particle diameter of a virus is desirable.

(c) A virus (e.g., HVJ, etc.) or an inactivated virus (e.g., HVJ, etc.) may be purified by column chromatography and then ultrafiltration, for example. For column chromatography, either a weak anion exchange material (an exchange group, such as DEAE (tertiary amine) or the like, is bound thereto) or a strong anion exchange material (an exchange group, such as QAE (quaternary amine) or the like, is bound thereto) may be used. Column chromatography using a gel filtration carrier may be employed.

A column is balanced in advance with about 3 bed volumes of buffered solution (pH 7.5, 150 mM NaCl). A solution of HVJ or inactivated HVJ having a pH of 7.5 is fed to the column. The column is washed with about 2 bed volumes of buffered solution (pH 7.5, 150 mM NaCl) and about 5 bed volumes of buffered solution (pH 7.5, 350 mM NaCl). Thereafter, the adsorbed HVJ or the adsorbed, inactivated HVJ is eluted with about 5 bed volumes of buffered solution (pH 7.5, 650 mM NaCl). A fraction having a peak absorption at 280 nm is recovered. Various buffered solutions may be used. The eluted fraction is condensed from 4-fold to 50-fold by ultrafiltration.

In a process of the present invention, a solution containing HVJ or inactivated HVJ may be subjected to filtration using a membrane filter (pore diameter: from 0.22 µm to 1.0 µm) before or after each step, if required.

An inactivated virus (e.g., HVJ, etc.) envelope of the present invention is useful as a reagent for preparing a gene Introduction vector. A gene introduction vector prepared with an inactivated virus (e.g., HVJ, etc.) envelope may be used for genetic function analysis, gene therapy, or the like.

### (Description of Preferred Embodiments)

The present invention can be carried out as follows.
(1) Inactivation without condensation of virus solution:
   (a) Step of inactivating a virus (e.g., HVJ, etc.): the virus is inoculated into chicken fertilized eggs, followed by proliferation of the virus. The chorioallantoic fluid is recovered. The virus is treated with an alkylating agent to inactivate the virus. Thereafter, the chorioallantoic fluid containing the inactivated virus is subjected to filtration.
   (b) Step of obtaining a condensate solution of the inactivated virus (e.g., HVJ, etc.): the filtrate is condensed by ultrafiltration.
   (c) Step of purification: the condensate solution of the inactivatedvirus is purified by column chromatography and then ultrafiltration. Further, ultrafiltration can be optionallyperformedtoadjust the inactivated virus envelope to a predetermined concentration.
(2) Inactivation after condensation of virus by high-speed centrifugation:
   (b) Step of obtaining a condensate solution of avirus (e.g., HVJ, etc.): the virus is inoculated into chicken fertilized eggs, followed by proliferation of the virus. The chorioallantoic fluid is recovered. Thereafter, as a pretreatment, the chorioallantoic fluid containing the virus is subjected to low-speed centrifugation to remove tissue pieces of the eggs. Further, high-speed centrifugation is performed. The supernatant is removed. The precipitate is suspended in buffered solution, which is a virus condensate solution. The virus condensate solution is preserved at 2°C to 6°C.
   (a) Step of inactivating the virus (e.g., HVJ, etc.): the virus is treated with an alkylating agent to inactivate the virus.
   (c) Step of purifying the inactivated virus: the inactivated virus is purified by column chromatography and ultrafiltration. Further, ultrafiltration can be optionallyperformed to ad just the inactivatedvirus envelope to a predetermined concentration.
(3) Inactivation after condensation of virus by density gradient centrifugation
   (b) Step of obtaining a condensate solution of a virus (e.g., HVJ, etc.): the virus is inoculated into chicken fertilized eggs, followed by proliferation of the virus. The chorioallantoic fluid is recovered. Thereafter, the chorioallantoic fluid containing the virus is subjected to low-speed centrifugation to remove tissue pieces of the eggs. Further, sucrose density gradient ultracentrifugation is performed. The virus on a sucrose solution layer is recovered. Sucrose is removed by dialysis. The virus is cyopreserved (at -40°C or -80°C).
   (a) Step of inactivatingthevirus (e.g., HVJ, etc.): the cryopreserved virus solution is thawed. The virus is treated with an alkylating agent to inactivate the virus.
   (c) Step of purifying the inactivated virus: the inactivated virus is purified by column chromatography and ultrafiltration. Further, ultrafiltration can be optionallyperformedto adjust the inactivatedvirus envelope to a predetermined concentration.
(4) Inactivation after condensation of virus by ultrafiltration:
   (b) Step of obtaining a condensate solution of avirus (e.g., HVJ, etc.): the virus is inoculated into chicken fertilized eggs, followed by proliferation of the virus. The chorioallantoic fluid is recovered. Thereafter, as a pretreatment, the chorioallantoic fluid containing the virus is subjected to filtration. The virus is subjected to ultrafiltration to obtain a virus condensate solution.
   (a) Stepof inactivating the virus (e.g., HVJ, etc.): the virus is treated with an alkylating agent to inactivate the virus.
   (c) Step of purifying the inactivated virus: the inactivated virus is purified by column chromatography and ultrafiltration. Further, ultrafiltration can be optionally performed to ad just the inactivated virus envelope to a predetermined concentration.
(5) Inactivation after purification of virus by column chromatography and then ultrafiltration:
   (c) Step of purification: the virus is inoculated into chicken fertilized eggs, followed by proliferation of the virus. The chorioallantoic fluid is recovered. Thereafter, the chorioallantoic fluid containing the virus is subjected to filtration. The virus is purified by column chromatography and then ultrafiltration.
   (a) Step of inactivating the virus (e.g., HVJ, etc.): the virus is treated with an alkylating agent to inactivate the virus.
   (b) Step of obtaining a condensate solution of the inactivated virus: the inactivated virus is condensed by ultrafiltration.
(6) In the case of influenza virus:
   An influenza virus used in a process according to a preferred embodiment of the present invention is, for example, obtained by culturing on a sensitive host cell, such as a mammalian cell (e.g., a kidney cell of a monkey, a hamster or a pig) or a cell of a ferret or a mouse, a cell derived embryo, a cell derived from human lung tissue, a cell derived from the fibroblast of a chick embryo, or the like.

A chicken embryo is the most commonly used system for production of industrial vaccines and is preferably used herein. Therefore, the present invention also relates to the above-described method for obtaining influenza viruses by culturing them in chicken embryos.

Fertilized eggs need to be carefully selected and obtained from specially secured healthy farms. The eggs are placed in an incubator at 37.8°C (100°F) for from 9 days to 12 days. The egg is held to the light of a candle to observe the growth or survival of the embryo before an influenza virus is inoculated into the allantois.

Thereafter, in order to infect the egg with the virus under optimal conditions, the egg is cultured for from 2 days to 3 days in a culture incubator having controlled temperature and humidity. The conditions vary depending on the line and type of the influenza virus used. The culture is rapidly cooled to 5±3°C to arrest the proliferation of the virus. Thereafter, allantois liquid containing a large amount of virus particles is recovered from the infected egg.

The thus-obtained allantois liquid containing the influenza virus needs to be rapidly purified to remove impurities, such as proteins (e.g., ovalbumin, etc.), lecithin, bacteria, and the like. To achieve this, the recovered material is centrifuged to remove the supernatant, followed by ultrafiltration to condense the material 20-fold before purification of the virus.

Techniques for purification of influenza viruses are well known to those skilled in the art, including separation methods, such as filtration, ultracentrifugation, affinity chromatography, and the like. By these operations, influenza viruses are condensed.

The envelope as prepared above can be prescribed as various compositions, pharmaceutical agents, agricultural chemicals by methods well known in the art, which are described in documents cited herein. Therefore, it is possible for those skilled in the art to use methods commonly used in the art based on the disclosure of the specification to prepare compositions in various forms intended by the present invention.

All patents, patent applications, journal articles and other references mentioned herein are incorporated by reference in their entireties.

The present invention has been heretofore described by illustrating preferred embodiments thereof. Hereinafter, the present invention will be described by way of examples. The above-described explanation and the examples described below are provided only for illustrative purposes and are not intended to limit the present invention. Therefore, the scope of the present invention is not limited by the embodiments and examples specified herein except as by the appended claims.

### (Examples)

Examples below are provided only for illustrative purposes. The present invention is not limited by the examples.

### (Example 1: Preparation of HVJ condensate solution)

### (1) Proliferation of HVJ

A seed HVJ virus was proliferated in SPF (Specific Pathogen Free) fertilized eggs, followed by isolation and purification. The resultant HVJ (Type Z) was dispensed into tubes for preserving cells. 10% DMSO was added to the tube which was in turn stored in liquid nitrogen.

Chicken eggs were obtained immediately after fertilization. The eggs were placed in an incubator (SHOWA-FURANKI P-03 type; which can accommodate about 300 chicken eggs can be accommodated), followed by incubation at 36.5°C, at a humidity of 40% or more, for from 10 to 14 days. In a dark place, an egg candler (a device which emits light of an electric lamp through a window having a diameter of about 1.5 cm) was used to confirm the survival of an embryo, and an air chamber and chorioallantois. A virus injection portion was marked about 5 mm above the chorioallantois with a pencil (except where thick blood vessels were observed). A polypeptone solution (1% polypeptone, 0.2% NaCl, 1 M NaOH, pH 7.2, autoclave sterilized, kept at 2°C to 6°C) was used to dilute a seed virus (removed from liquid nitrogen) 500-fold. The resultant solution was placed at 2°C to 6°C. The egg was sterilized with isodine and alcohol. A small hole was made at the virus injection portion using an awl, and 0.1 mL of the diluted seed virus was injected into the chorioallantoic cavity using a 1-mL syringe with a 26-gauge needle. Melted paraffin (melting point: from 50°C to 52°C) was placed on the hole using Pasteur's forceps to close the hole. The egg was placed in an incubator at from 34°C to 36.5°C, at a humidity of 40% or more, and for 3 days. Thereafter, the inoculated egg was placed at from 2°C to 6°C overnight. On the following day, the air chamber portion of the egg was broken with forceps. A 10-mL syringe with a 18-gauge needle was inserted into the chorioallantois to suction and collect chorioallantoic fluid into a sterilized bottle, which was preserved at from 2°C to 6°C.

### (2) Condensation of HVJ

About 100 mL of the above-described HVJ-containing chorioallantoic fluid obtained in the step (1) of Example 1 (chorioallantoic fluid collected from HVJ-containing chicken eggs and preserved at from 2°C to 6°C) was dispensed into two about 50-mL centrifugation tubes using a widemouthed Komageme type pipette, followed by centrifugation using a low-speed centrifuge at 3,000 rpm, for 10 minutes, at from 2°C to 6°C (brake: off) to remove tissue pieces of the egg.

After centrifugation, the supernatant was dispensed into four 35-mL centrifugation tubes (for high-speed centrifugation), followed by centrifugation using an angle rotor at 27,000×g for 30 minutes (accelerator and brake: on). The supernatant was removed. BSS (10 mM Tris-HCl (pH 7.5), 137 mM NaCl, 5.4 mM KCl; autoclave sterilized, preserved at from 2°C to 6°C) was added to the precipitate (PBS is substitutable for BSS) in an amount of about 5 mL per tube. The tube was allowed to stand at from 2°C to 6°C. The precipitate was broken up using a widemouthed Komageme type pipette and was collected into a tube, followed by centrifugation using an angle rotor at 27,000×g for 30 minutes. The supernatant was removed. About 10 mL of BSS was added to the precipitate. The tube was allowed to stand at from 2°C to 6°C. The precipitate was broken up using a widemouthed Komageme type pipette, followed by centrifugation using a low-speed centrifuge at 3,000 rpm for 10 minutes at from 2°C to 6°C (brake: off). Tissue pieces or virus agglutinates which had not been removed were removed. The supernatant was placed in a new sterilized tube and preserved at from 2°C to 6°C as an HVJ condensate solution.

0.9 mL of BSS was added to 0.1 mL of the HVJ condensate solution. The absorbance at 540 nm of the mixture was measured using a spectrophotometer. Virus titer was converted to red blood cell agglutination activity (HAU). An absorbance at 540 nm of 1 substantially corresponds to 15,000 HAU. It is considered that HAU is substantially proportional to fusion activity.

### (Example 2: Preparation of HVJ condensate solution)

Further, HVJ may be optionally purified using sucrose density gradient. Specifically, the HVJ suspension obtained in Example 1 was placed on a centrifugation tube in which 60% and 30% sucrose solutions (sterilized) were layered, followed by density gradient centrifugation at 62,800×g for 120 minutes. After centrifugation, a band observed on the 60% sucrose solution layer was recovered. The recovered HVJ suspension was subjected to dialysis with BSS or PBS as external dialysis buffer at from 2°C to 6°C overnight to remove sucrose. Glycerol (autoclave sterilized) and 0.5 M EDTA solution (autoclave sterilized) were added to the HVJ suspension to a final concentration of 10% and from 2 mM to 10 mM, respectively. The mixture was mildly frozen at -80°C, and finally preserved in liquid nitrogen, when it is not immediately used (cryopreservation can be carried out with 10 mM DMSO instead of glycerol and 0.5 M EDTA solution).

### (Example 3: Inactivation of HVJ with alkylating agent)

Immediately before use, 0.01% β-propiolactone was prepared in 10 mM KH₂PO. This procedure was rapidly performed at low temperature.

β-propiolactone was added to the HVJ condensate solution obtained in Example 1, followed by incubation on ice for 60 minutes. Thereafter, incubation was performed at 37°C for 2 hours. The resultant solution was dispensed into Eppendorf tubes at 10,000 HAU per tube, followed by centrifugation at 15,000 rpm for 15 minutes. The precipitate was preserved at -20°C.

### (Example 4: Preparation of inactivated HVJ by column chromatography and then ultrafiltration)

After collection, the chorioallantoic fluid obtained in step (1) of Example 1 was subjected to filtration using from 80 µm to 10 µm nylon mesh filter. 0.006% to 0.008% β-propiolactone (final concentration) was added to the chorioallantoic fluid (2°C to 6°C, 1 hour) to inactivate HVJ. The chorioallantoic fluid was incubated at 37°C for 2 hours to inactivate β-propiolactone.

Ultrafiltration using 500 KMWCO (A/G Technology, Needham, Massachusetts) was used to condense the chorioallantoic fluid about 10-fold. 50 mMNaCl, 1 mM MgCl₂, 2% mannitol, 20 mM Tris (pH 7.5) was used as buffered solution. HA assay was used to achieve an HVJ recovery rate of substantially 100%. This is an excellent effect.

Column chromatography was performed using QSepharoseFF (Amersham Pharmacia Biotech K.K., Tokyo) (buffered solution: 20 mM Tris-HCl (pH 7.5) buffer, from 0.2 M to 1 M NaCl)) to purify HVJ. As a result, the recovery rate was from 40% to 50%, and the purity was 99% or more.

HVJ was condensed by ultrafiltration using 500KMWCO (A/G Technology).

### (Example 5: Inactivation of HVJ with alkylating agent)

300 mL of a condensed and frozen product obtained in substantially the same manner as in Example 1 was thawed at from 34°C to 35°C, and was supplemented with an antibiotic. The product was immersed in a water bath at 22°C for 30 min. Thereafter, 24 µL of β-propiolactone (purity: 90% or more, produced by Sigma) was added to the product, followed by immersion of the product in a water bath at 22°C for 1 hour and then in a water bath at 37°C for 2 hours. The inactivating procedure was completed to obtain an inactivated HVJ condensate solution.

### (Example 6: Preparation of inactivated HVJ by column chromatography and then ultrafiltration)

### (1) Preparation by column chromatography

The inactivated HVJ condensate solution obtained in Example 5 was fed into Q-Sepharose FF column (diameter: 20 cm, bed height: 15 cm, bed volume: 4710 mL) balanced with 15 L of buffered solution 1 (20 mM Tris-HCl (pH 7.5), 150 mM NaCl) at a flow rate of 50 mL/min. Thereafter, 10 L of buffered solution 1 (20 mM Tris-HCl (pH 7.5), 150 mM NaCl) , and 25 L of buffered solution 2 (20 mM Tris-HCl (pH 7.5), 350 mM NaCl) were passed through the column in sequence. The inactivated HVJ was adsorbed into a column resin when the condensate solution was fed to the column, while most impurities in the inactivated HVJ condensate solution were washed off the resin with the buffered solutions 1 and 2. 25 L of buffered solution 3 (20 mM Tris-HCl (pH 7.5), 650 mM NaCl) was passed through the column and HVJ was substantially concurrently eluted from the resin and collection of a column fraction was started. 7829 mL of a fraction was obtained from a time when a peak of inactivated HVJ appeared on an UV absorption chart (λ=280 nm) until the level of inactivated HVJ returned to the base line. An antibiotic was added to the fraction. After obtaining the fraction, passing of the buffered solution was continued, and finally, 20 L of buffered solution 4 (20 mM Tris-HCl (pH 7.5), 1 M NaCl) was passed through the column.

### (2) Preparation by ultrafiltration

The column fraction obtained in step (1) of Example 6 was placed in a 10-L bottle. A cap having an attached supply tube and circulation tube was put on the bottle. The supply tube was connected via a Perista pump to the inlet of UFP-500-E-5A ultrafiltration module (produced by A/G Technology Corporation). The circulation tube was connected via a circulation amount regulating valve to the outlet of the module. The pump was operated so that the circulation amount regulating valve was throttled to perform condensation and discharged drainage at from 60 mL/min to 70 mL/min while the outlet pressure of the module was kept at from 40 kPa to 80 kPa.

When the amount of the circulating solution reached about 600 mL, the bottle was exchanged with a 500-mL bottle while the module was exchanged with UFP-500-E-4A (produced by A/G Technology Corporation). Then, condensation was continued. In a manner similar to that described above, drainage was discharged at about 10 mL/min. When the amount of the circulating solution reached about 60 mL, 60 mL of buffered solution 5 (20 mM Tris-HCl (pH 7.5), 50 mM NaCl, 1 mM MgCl₂, 2% mannitol) was added, and condensation was further continued until the amount of the circulating solution reached about 60 mL (buffer exchange). Further, buffer exchange was performed two times. Thereafter, the amount of the circulating solution reached 79 mL. The circulating solution was taken into a 5 mL disposal syringe. A disc filter (Sterile Syringe Filter produced by CORNING, φ=26 mm, 0.45 µm) was attached to the tip of the syringe. Sterile filtration was carried out manually. 65 mL of inactivated HVJ envelope was finally obtained.

### (Example 7: Production of inactivated HVJ envelope from HVJ-containing chorioallantoic fluid)

### (1) Inactivation of HVJ with alkylating agent

An antibiotic was added to 6150 mL of HVJ-containing chorioallantoic fluid obtained in a manner similar to that of step (1) in Example 1. The mixture was immersed in a water bath at 22°C. 492 µL of β-propiolactone (purity: 90% or more, produced by Sigma) was added to the mixture, followed by immersion of a water bath at 22°C for 1 hour and then a water bath at 37°C for 2 hours. The inactivating method was completed and inactivated HVJ-containing chorioallantoic fluid was obtained.

### (2) Pretreatment (filtration)

6150 mL of the inactivated HVJ-containing chorioallantoic fluid obtained in step (1) of Example 7 was supplied via a Perista pump to a cartridge filter (Polygard-CR cartridge filter produced by Millipore, 5 µm) for filtration. After filtration, 300 mL of buffered solution 1 (20 mM Tris-HCl (pH 7.5), 50 mM NaCl, 1 mM MgCl₂, 2% mannitol) was added to wash the pipe or the filter. The final filtrated chorioallantoic fluid had a volume of 6500 mL.

### (3) Condensation by ultrafiltration

6500 mL of the chorioallantoic fluid filtrate obtained in step (2) of Example 7 was placed in a 10-L bottle. A cap having an attached supply tube and circulation tube was put on the bottle. The supply tube was connected via a Perista pump to the inlet of an UFP-500-E-6A module (produced by A/G Technology Corporation). The circulation tube was connected via a circulation amount regulating valve to the outlet of the module. The pump was operated. The circulation amount regulating valve was throttled to perform condensation while the module outlet pressure was kept at 40 kPa to 100 kPa. In this case, drainage was discharged at 80 mL/min to 200 mL/min. When visual inspection confirmed that the amount of the circulating solution reached about 650 mL, 650 mL of buffered solution 1 (step (1) of Example 7) was added. Further, condensation was continued until the amount of the circulating solution reached about 650 mL (Buffer exchange). Further, Buffer exchange was performed two times. As a result, the amount of the circulating solution as a condensate solution reached 780 mL.

### (4) Purification by column chromatography

The inactivated HVJ condensate solution obtained in step (3) of Example 7 was fed into Q-Sepharose FF column (diameter: 20 cm, bed height: 15 cm, bed volume: 4710 mL) balanced with 15 L of buffered solution 2 (20 mM Tris-HCl (pH 7.5), 150 mM NaCl) at a flow rate of 50 mL/min. Thereafter, 10 L of buffered solution 1 (20 mM Tris-HCl (pH 7.5), 150 mM NaCl), and 25 L of buffered solution 3 (20 mM Tris-HCl (pH7.5), 350 mM NaCl) were passed through the column in sequence. The inactivated HVJ was adsorbed into a column resin when the condensate solution was fed to the column, while most impurities in the inactivated HVJ condensate solution were washed off the resin with buffered solutions 2 and 3. 25 L of buffered solution 4 (20 mM Tris-HCl (pH 7.5), 650 mM NaCl) was passed through the column and HVJ was substantially concurrently eluted from the resin and collection of a column fraction was started. 10800 mL of a fraction was obtained from a time when a peak of inactivated HVJ appeared on an UV absorption chart (λ=280 nm) until the level of inactivated HVJ returned to the base line. An antibiotic was added to the fraction. After obtaining the fraction, passing of the buffered solution was continued, and finally, 20 L of buffered solution 5 (20 mM Tris-HCl (pH 7.5), 1 M NaCl) was passed through the column.

### (5) Preparation by ultrafiltration

The column fraction obtained in step (4) of Example 7 was placed in a 10-L bottle. A cap having an attached supply tube and circulation tube was put on the bottle. The supply tube was connected via a Perista pump to the inlet of a module comprising two UFP-500-E-5A ultrafiltration modules in series (produced by A/G Technology Corporation). The circulation tube was connected via a circulation amount regulating valve to the outlet of the module. The pump was operated so that the circulation amount regulating valve was throttled to perform condensation and discharged drainage at from 40 mL/min to 60 mL/min while the outlet pressure of the module was kept at from 40 kPa to 80 kPa. When the amount of the circulating solution reached about 700 mL, the bottle was exchanged with a 1-L bottle. Then, condensation was continued.

When the amount of the circulating solution reached about 200 mL, 300 mL of buffered solution 1 (step (2) of Example 7) was added. Further, condensation was continued until the amount of the circulating solution reached about 200 mL (Buffer exchange). Further, Buffer exchange was performed two times. As a result, the amount of the circulating solution as a condensate solution reached 300 mL (inactivated HVJ envelope).

### (Example 8: Measurement of the amount of genes introduced with inactivated HVJ envelope using luciferase gene)

BHK-21 (child Syrian hamster kidney cell) (ATCC No. CCL-10, purchased from Dainippon Pharmaceutical) was suspended in Basal Medium Eagle (Sigma, No. B-1522) culture medium supplemented with 10% fetal calf serum and 10% tryptose phosphate broth (Dainippon Pharmaceutical, No. 16-821-49) to 2.5×10⁴ cells/0.5 mL/well (24-well plastic plate), followed by culture in an incubator at 37°C in 5% carbon dioxide gas. After 20 to 24 hours culture, the amount of a gene introduced with an inactivated HVJ envelope was measured as follows.

5 µL of 2 mg/mL protamine sulfate solution (PBS) obtained in a manner similar to that of Example 7 was addd to 20 µL of Inactivated HVJ envelope suspension, followed by mixing. The mixture was allowed to stand on ice for 5 minutes. Thereafter, 5 µL (10 µg) of solution containing plasmid DNA (pGL3) encoding a luciferase gene was added to the mixture, followed by mixing. Further, 3 µL of 2% Triton X-100 (PBS(-)) was added to the mixture, followed by mixing. The mixture was centrifuged at 15000 rpm (19500×g) at from 2°C to 6°C for from 10 min to 15 min.

After removal of the supernatant, the precipitate was suspended in 30 µL of PBS(-). 5 µL of 1 mg/mL protamine sulfate solution (PBS) was added to the suspension, followed by mixing. 8 µL (per well) of the mixture was added to the previously prepared (cultured) BHK-21 cells.

From 20 hours to 24 hours after addition, the expression level of luciferase was measured using a luciferase measurement kit (LucLite, No. 6016911, produced by Packard). The amount of light emission was measured using a LUMINOMETER (TD-20e, produced by Turner). PBS was Dulbecco's Phosphate Buffer Saline (No. D-8662, Sigma).

As a result, it was demonstrated that HVJ envelopes can be used to introduce a biological macromolecule, such as a gene or the like.

### (Example 9: Use of influenza virus)

### (1) Preparation of influenza virus:

An influenza virus of the family Orthomyxovirus is basically obtained from chicken embryos as described in, for example, WO96/05294, followed by proliferation. Briefly, fertilized eggs need to be carefully selected and obtained from specially secured healthy farms. The eggs are placed in an incubator at 37.8°C (100°F) for from 9 to 12 days. The egg is held to the light of a candle to observe the growth or survival of the embryo before an influenza virus is inoculated into the allantois.

Thereafter, in order to infect the egg with the virus under optimal conditions, the egg is cultured for from 2 to 3 days in a culture incubator having controlled temperature and humidity. The conditions vary depending on the line and type of the influenza virus used. The culture is rapidly cooled to 5±3°C to arrest the proliferation of the virus. Thereafter, allantois liquid containing a large amount of virus particles is recovered from the infected egg.

The thus-obtained allantois liquid containing the influenza virus needs to be rapidly purified to remove impurities, such as proteins (e.g., ovalbumin, etc.), lecithin, bacteria, and the like. To achieve this, the recovered material is centrifuged to remove the supernatant, followed by ultrafiltration to condense the material 20-fold before purification of the virus.

### (Alkylation)

As described in Example 3, the thus-prepared influenza virus is inactivated. As described in Example 4, ultrafiltration is performed.

Thereafter, as described in Example 5 the influenza virus is inactivated. Thereafter, as described in Example 6, the influenza virus is subjected to ultrafiltration.

Further, as described in Example 7, an inactivated influenza virus envelope is produced from influenza virus-containing chorioallantoic fluid.

### (Example 10: Measurement of the amount of genes introduced with inactivated influenza virus envelope using luciferase gene)

An inactivated influenza virus envelope suspension obtained in Example 9 and a protocol similar to that described in Example 8 are used to measure the amount of introduced genes. As a result, it is found that the luciferase gene was introduced as with HVJ.

Therefore, it is demonstrated that an influenza virus can be used as a safe vector for introducing a biological molecule in the present invention.

### INDUSTRIAL APPLICABILITY

According to the process of the present invention, viruses (e.g., HVJ, etc.) can be inactivated uniformly and efficiently as compared to conventional methods. In addition, the proliferation ability of the inactivated virus (e.g., HVJ, etc.) is inactivated, while the fusion activity of the envelope of the virus (e.g., HVJ, etc.) can be retained. Therefore, it is advantageous that appropriate inactivated virus (e.g., HVJ, etc.) envelopes can be industrially produced.

## Claims

1. A process for producing an inactivated virus envelope, comprising the step of:
inactivating a virus with an alkylating agent.

2. A process for producing an inactivated virus envelope, comprising the steps of:
(a) inactivating a virus with an alkylating agent;
(b) obtaining a condensate solution of the virus or the inactivated virus; and
(c) purifying the virus or the inactivated virus by column chromatography and then ultrafiltration.

3. A process according to claim 2, comprising the steps of:
(a) inactivating a virus with an alkylating agent;
(b) obtaining a condensate solution of the virus or the inactivated virus; and
(c) purifying the virus or the inactivated virus by column chromatography and then ultrafiltration,
wherein the steps are conducted in this order.

4. A process according to claim 2, comprising the steps of:
(b) obtaining a condensate solution of a virus;
(a) inactivating the condensed virus with an alkylating agent; and
(c) purifying the inactivated virus by column chromatography and then ultrafiltration,
wherein the steps are conducted in this order.

5. A process according to claim 2, comprising the steps of:
(c) purifying a virus by column chromatography and then ultrafiltration;
(a) inactivating the purified virus with an alkylating agent; and
(b) obtaining a condensate solution of the inactivated virus,
wherein the steps are conducted in this order.

6. A process according to any one of claims 2, 3, 4, and 5, wherein the step of obtaining the condensate solution of the virus or the step of obtaining the condensate solution of the inactivated virus comprises performing centrifugation.

7. A process according to any one of claims 2, 3, 4, and 5, wherein the step of obtaining the condensate solution of the virus or the step of obtaining the condensate solution of the inactivated virus comprises performing ultrafiltration.

8. A process according to claim 1 or 2, wherein the virus is Sendai virus or influenza virus.

9. A process according to claim 1 or 2, wherein the virus is Sendai virus.

10. A process for producing a composition comprising a viral envelope, comprising the steps of:
(a) inactivating a virus with an alkylating agent;
(b) obtaining a condensate solution of the virus or the inactivated virus; and
(c) purifying the virus or the inactivated virus by column chromatography and then ultrafiltration;
wherein the steps are conducted in any order, and subsequently,
(d) mixing the purified inactivated virus with a material to be introduced therewith.

11. A process according to claim 10, wherein the material is a biological macromolecule.

12. A process according to claim 10, wherein the material is selected from the group consisting of nucleic acids, polypeptides, sugars, lipids, and complexes thereof.

13. A process according to claim 10, wherein the virus is Sendai virus or influenza virus.

14. A process according to claim 10, wherein the virus is Sendai virus.

15. A process for producing a medicament comprising a viral envelope, comprising the steps of:
(a) inactivating a virus with an alkylating agent;
(b) obtaining a condensate solution of the virus or the inactivated virus; and
(c) purifying the virus or the inactivated virus by column chromatography and then ultrafiltration;
wherein the steps are conducted in any order, and subsequently,
(d) mixing the purified inactivated virus with a medical ingredient to be introduced therewith.

16. A process according to claim 15, wherein the medical ingredient is a biological macromolecule.

17. A process according to claim 15, wherein the medical ingredient is selected from the group consisting of nucleic acids, polypeptides, sugars, lipids, and complexes thereof.

18. A process according to claim 15, wherein the medical ingredient is a nucleic acid encoding a polypeptide expressed in a host to which the nucleic acid is introduced.

19. A process according to claim 15, wherein the medical ingredient is in the form of a vaccine.

20. A process according to claim 15, wherein the virus is Sendai virus or influenza virus.

21. A process according to claim 15, wherein the virus is Sendai virus.

22. A composition obtained by a process according to any one of claims 10 to 14.

23. A medicament obtained by a process according to any one of claims 15 to 21.
